# EUROPEAN PATENT SPECIFICATION

(11) **EP 1 410 330 B1**
(45) Date of publication and mention of the grant of the patent: **29.08.2007**
(21) Application number: 02750684.9
(22) Date of filing: 16.07.2002
(51) Int. Cl.: G06T 5/00

(54) **Simultaneous aqusition of different time-gates in TPSF-based optical imaging**
Simultane Aufnahme verschiedener Zeitfenster bei zeitauggelöster optischer Streulichtspektroskopie
Enregistrement simultane de fenêtre temporelles distinctés pour imagerie optiques par une fonction d'êtalement de points temporeles

(30) Priority: 16.07.2001 US 305081 P
(43) Date of publication of application: 21.04.2004
(73) Proprietor: ART Advanced Research Technologies Inc., St-Laurent Quebec, H4S 2A4 (CA)
(72) Inventor: HALL, David Jonathan, Montréal, Québec H3G 1X9 (CA)
(74) Representative: Joly, Jean-Jacques
(86) International application number: PCT/CA2002/001067
(87) International publication number: WO 2003/009229

(56) References cited:
- WO-A-99/27343
- US-A- 4 958 231
- SCHMIDT F E W ET AL: "A 32-CHANNEL TIME-RESOLVED INSTRUMENT FOR MEDICAL OPTICAL TOMOGRAPHY" REVIEW OF SCIENTIFIC INSTRUMENTS, AMERICAN INSTITUTE OF PHYSICS. NEW YORK, US, vol. 71, no. 1, 1 January 2000 (2000-01-01), pages 256-265, XP001006491 ISSN: 0034-6748

## Description

### TECHNICAL FIELD

This invention relates to the field of temporal point spread function (TPSF) based imaging in which objects which diffuse light, such as some human body tissues, are imaged using signals resulting from the injection of light into the object and detection of the diffusion of the light in the object at a number of positions while gathering TPSF-based data to obtain information beyond simple attenuation such as scattering and absorption. More particularly, the present invention relates to a specific data-type, N-time-gate, which can be obtained from TPSF-based data.

### BACKGROUND OF THE INVENTION

Time-domain optical medical imaging shows great promise as a technique for imaging breast tissue, as well as the brain and other body parts. The principle of the technique lies in the analysis of the temporal point spread function (TPSF) of an injected pulse of light which is diffused in an object of interest (OOI), and in the extraction of information from the TPSF to construct a medically useful image.

Specific types of data extracted from the TPSF are known as 'Data-types'. For example, 'early time-gated attenuation' is a Data-type that can be extracted from the TPSF which improves the image spatial resolution over previous continuous wave methods. However, it is unclear whether such improvements in image spatial resolution are adequate for diagnosing certain diseases, such as breast cancer, based on morphology.

An alternative approach is to use the TPSF to decouple the light attenuation into absorption and scattering components. This extra information, which cannot be obtained from continuous wave methods, may be clinically useful. In order to achieve this it is necessary to extract appropriate Data-types from the TPSF. Researchers, in the time-domain field, have looked at data-types such as the meantime and higher moments of the TPSF. Whilst data-types for TPSF-based data acquired in the frequency domain have included phase-shift and amplitude.

There are several techniques for measuring the TPSF arising from a light pulse that has traveled through an 001 such as breast tissue. Certainly the most complete time-domain information one can acquire is to measure the complete TPSF. This TPSF-based information can also be acquired in the frequency domain, although current hardware limitations mean that time-domain hardware is capable of acquiring information over a larger bandwidth than its frequency domain counterpart.

Published Application No. WO 99/27343 discloses a breast imaging device using a photodetector to measure a laser pulse diffused by passing through breast tissue.

However, acquiring the complete TPSF has implications in terms of long acquisition times for clinical systems, plus long data-processing for image reconstruction algorithms.

It would therefore be desirable to provide methods overcoming the limitations of the prior art.

### SUMMARY OF THE INVENTION

For the purpose of image construction, the entire TPSF derived from a pulse of light can be used. The streak camera has commonly been used for this purpose. However the detection surface of a streak camera may be limited. Time-gated cameras such as intensified charge coupled device (ICCD) typically have greater detection surface areas but acquisition of complete TPSF's using these camera may be time consuming.

It is therefore an object of the present invention to provide a method and apparatus for TPSF-based imaging of an object in which acquisition times are reduced while still extracting a sufficient amount of desired information from the TPSF of a collected optical signal to be used in producing an optical image.

According to a broad aspect of the invention this objective is achieved by detecting selected intervals, or N-time-gates, of a TPSF. The detection of the selected time-gates is effected using time-gated detection techniques and cameras.

In a further aspect of the invention, the acquisition time is substantially reduced by simultaneously detecting selected time gates of a TPSF.

Thus in one aspect of the present invention there is provided a method for simultaneously detecting N-time-gates Data-type of a TPSF of a collected optical signal, the method comprising pulsing the object with a light pulse delivered at one or more injection ports, collecting a plurality of optical signals based TPSF⁻¹s at collection ports, introducing delays in propagation of the optical signals to produce staggered TPSF's and simultaneously detecting selected time-gates.

In an embodiment of the invention the delays in the propagation of the optical signals are provided by using optical fibers of different lengths to collect the optical signals.

### BRIEF DESCRIPTION OF THE DRAWINGS

Further features and advantages of the present invention will become apparent from the following detailed description, taken in combination with the appended drawings, in which:
Fig. 1 is a diagram showing an embodiment of the system used to generate a pulse of light and to produce a TPSF after injection of the light in an object;
Fig. 2 is a graphical representation of a TPSF showing arbitrarily defined time-gates;
Fig. 3 is an example of a system that can be used to construct an optical image according to methods of the present invention;
Fig. 4 is a schematic representation of an embodiment of the system used to simultaneously detect two or more time-gates; and
Fig. 5 is a graphical representation of three staggered TPSF's.

It will be noted that throughout the appended drawings, like features are identified by like reference numerals.

### DETAILED DESCRIPTION OF THE PREFERRED EMBODIMENT

In accordance with the present invention, there is provided a method and apparatus using a N-time-gate Data-Type for TPSF-based optical imaging.

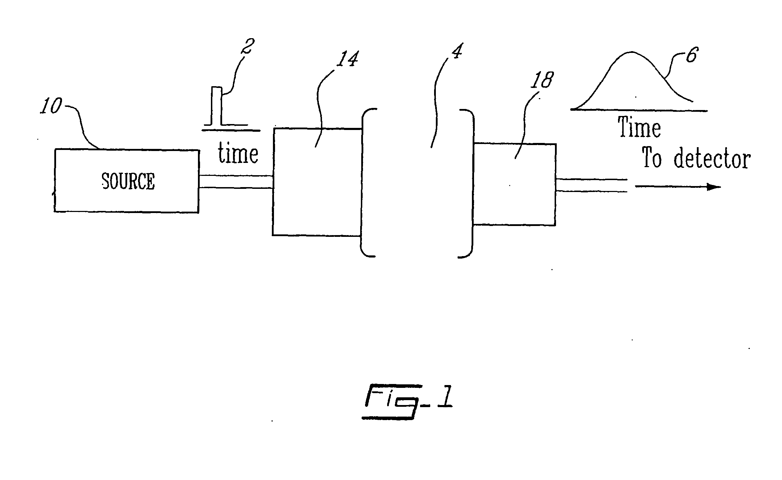

Figure 1 schematically represents the events giving rise to a TPSF. A pulse of light 2 is generated at a source 10 and injected in an OOI 4 at injection port 14. The OOI may be a body part such as a breast but it will be appreciated that the method and apparatus described herein can be applied to any suitable object. The light interacts with the medium by being absorbed and/or scattered and exits the OOI with an attenuated intensity. The exiting attenuated light can be collected at collection port 18 as a function of time to produce the TPSF 6. The shape of the TPSF depends on the type of interactions that occurred between the light and the medium. Images may be constructed by extracting appropriate Data-types from the TPSF and by inputting them to an appropriate inversion image reconstruction algorithm, i.e. one which forward models the data embedded within a minimization inversion loop.

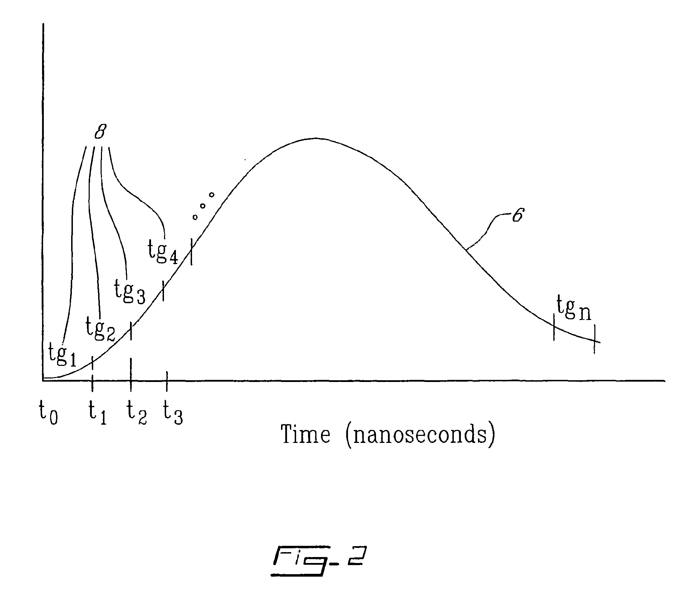

In one embodiment, the present invention provides a method for detecting only selected intervals of a TPSF. In Figure 2 it can be seen that a TPSF can be subdivided in n arbitrary intervals 8 which will be referred to as time-gates (*tg*) hereinafter. Therefore one can consider a complete TPSF as being constructed from many individual time-gate signals. The actual time points that defines the beginning and the end of a time-gate are preferably determined relative to the time, tₒ, at which the light pulse is injected into the object. By detecting the signal of sequential time-gates using time gated detection techniques and cameras such as, but not limited to, a time-gated intensified charge coupled device (ICCD), it is possible to acquire the complete TPSF. However, acquisition of a complete TPSF in this manner requires long acquisition times which will be proportional to the number of time-gates acquired. In one aspect of the present invention the image may be constructed by acquiring only certain time-gates. As a result the acquisition time is greatly reduced.

The selection of the time-gates relative to tₒ that will be used to construct an image may be based on the characteristics of the object such as the thickness and the anticipated optical properties. In the absence of prior knowledge about the anticipated optical properties of the object, a complete TPSF could be obtained and be used for future selection the time-gates.

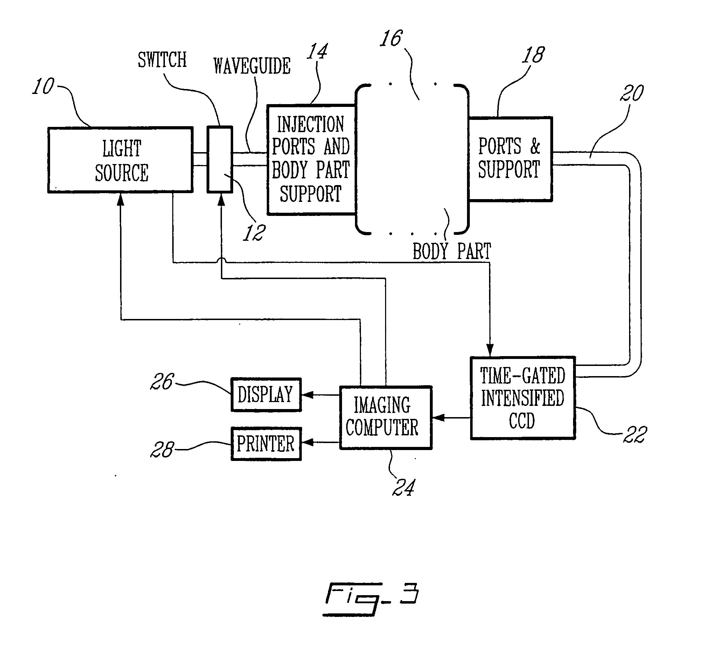

An embodiment of the system that can be used to perform the method of the instant invention is schematically represented in Figure 3. The pulsed light source 10 has an output optically coupled via a switch 12 to one or a plurality of injection ports 14 of a support. The injections port are preferably positioned at a number of fixed positions over the imaging area although the injection ports may alternatively be moveable over the body surface, provided that the body part 16 being imaged is immobilized. As is known in the art the injection and collection ports may directly contact the body or a coupling medium may be used between the body and the injection/collection ports. In Figure 3, the collection ports 18 and support are arranged in transmission mode for breast imaging. However, it is also possible to have an injection ports/object/collection ports geometry such that the collection ports are on the same surface as the injection ports. The light source may be a polychromatic source or a monochromatic source such as a laser. The pulses preferably have a duration of about 1 to 100 picoseconds and an average power of 100 mW.

The collected optical signals may be communicated by one or more waveguides 20 such as 400/440 micrometer graded index multimode optical fibers to one or more detection positions of a time gated detector 22 such as an ICCD camera, for example a PicoStar camera by LaVision.

A large number of pulses may be injected and their corresponding camera signals detected and processed by imaging computer 24 to determine the value of at least one time gate and, in the case of simultaneous detection, two or more selected time gates of a TPSF. Such data are gathered for a sufficiently large number of detecting port positions to construct an image of the object. The resulting image can be displayed on a monitor 26 or a hard copy can be obtained at 28.

It will be appreciated that the relative positions of the injectors and detectors may be modified as needed to optimize the quality of the image. Thus different geometries of the injection ports/collection ports/object assembly may be used, for example by placing collection ports either proximally or distally or a combination of both, depending on several factors such as, the thickness of the object, the nature of the medium comprising the object. The value for each pixel or voxel of an image generated from the data collected according to the present invention will benefit from a plurality of source detector locations with respect to the position of the pixel or voxel, as well as a plurality of time-gates.

In an aspect of the present invention the acquisition time can be further reduced by simultaneously detecting a desired number of time-gates of one or more TPSF's. This can be achieved by collecting the light signal exiting from the OOI at several (*m*) locations. Temporal delays are then introduced in the propagation of these optical signals such that the *m* TPSF's reach the time-gated detector in a staggered manner. Therefore, using a time-gated camera, it is possible to simultaneously detect *m* different time-gates of the TPSF's.

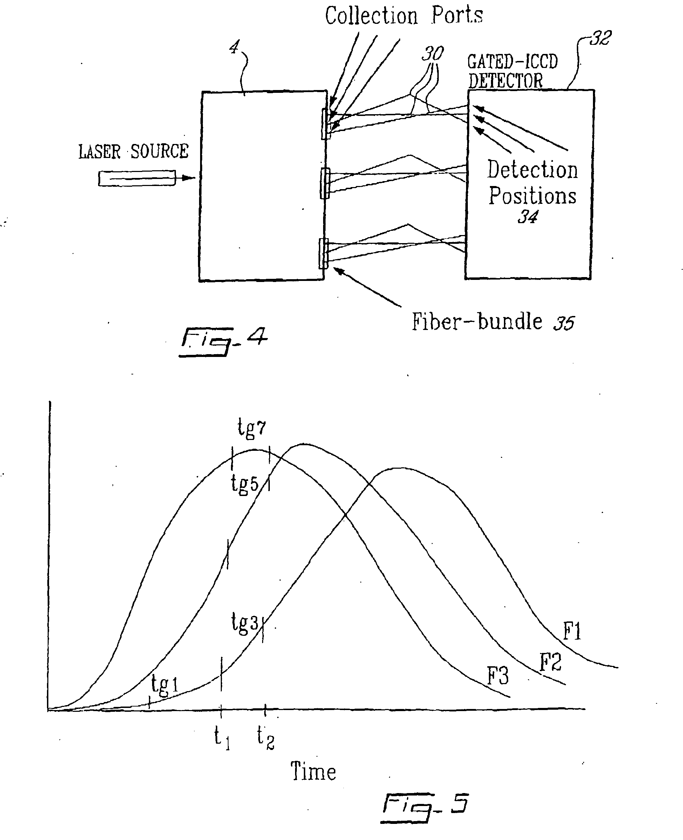

This embodiment of the invention will be better understood with reference to Figures 4 and 5. In Figure 4 a schematic diagram of a possible arrangement of a system used to simultaneously detect *m* time gates is depicted. In this embodiment optical fibers 30 are used to collect and channel the light exiting the OOI 4 to a time-gated detector 32. The fibers are of different lengths and therefore the time required for the optical signal to travel from the collecting point to the detection point at the detector is different for each fiber thus introducing delays in the propagation of the optical signal. Each fiber, or bundle of fibers, is coupled to a different detection position 34 at the detector. Consequently, the time required for a particular time gate, *tg*, to reach the detection position in the detector will depend on the length of the fiber through which it is propagated. Figure 5 is a graphic representation of three staggered TPSF's propagated by three different optical fibers F1, F2, and F3 having different lengths with length of F1> length of F2> length of F3. It can be appreciated that a given time-gate, say *tg*3, reaches the detector at a different time depending on whether it is propagated by F1, F2 or F3. Now if the detector is time-gated to detect only the signal during the interval defined by t1 and t2, different time-gates will be detected for each fiber. In our example *tg*3, *tg*5, and *tg*7 are simultaneously detected. It can be seen that the number of time gates that can be detected simultaneously is equal to the number of fibers. The fibers can be bundled within a defined area of a given detector and provide the desired time-gates simultaneously with one 'snapshot' of the time-gated camera. This leads to a decrease in the acquisition time by a factor of approximately n compared to serially acquired time-gates.

It will be appreciated that the length of the fibers can be selected to detect the desired time gates (or the desired sections of the TPSF). To refer to our example, if one wanted to simultaneously detect tg7, tg5 and tg1, the length of F1 could be increased accordingly. However, it will also be appreciated that the simultaneous detection of the desired time-gates can be effected by directing each fiber to a different detector and by adjusting the time gate of each detector so as to detect one of the desired time gate. Typically, an increase of 11 -cm in fiber length will induce a time delay of about 500 ps.

The delays in the propagation of the optical signals may also be introduced by using variable delay optical waveguides that are well known in the art.

It will be appreciated that the signal to noise ratio (SNR) may limit the number of fibers and thus the number of time gates that can be simultaneously detected. In practice, it is a decision between how many detector positions are required, the number of time-gates simultaneously required and the total number of fibers which can be packed onto the gated camera. Referring back to Figure 4, it will be further appreciated that if the fibers or the fiber bundles 35 are sufficiently close together, the TPSF's may be substantially identical.

The embodiment(s) of the invention described above is(are) intended to be exemplary only. The scope of the invention is therefore intended to be limited solely by the scope of the appended claims.

## Claims

1. A method for reducing image acquisition time when optically imaging a light-scattering object, the method comprising steps of:
i) injecting a pulse of light at an injection port into said object at a time tₒ;
ii) collecting light from said object at at least two collection ports to provide a plurality of optical signal-based temporal point spread functions; and
iii) detecting at least a first time gate of each said temporal point spread function in order to provide information to be used in producing an optical image of said light scattering object;
**characterized in that** desired temporal delays are introduced in the propagation path of the optical signals to produce time-delayed temporal point spread functions, wherein each of said first time-gates is obtained from a different time-delayed temporal point spread function, and wherein all of said first time-gates are simultaneously detected.

2. The method as claimed in claim 1 wherein said two or more collection ports are proximal and said temporal point spread functions are substantially identical.

3. The method as claimed in any one of claims 1 - 2 wherein said time-gates span a time interval defined by an initial time and a final time which are set relative to tₒ.

4. The method as claimed in claim 3 wherein said selected time gates are used for a plurality of injection port/object/detector port geometries.

5. The method as claimed in any one of claims 1 - 4 wherein said time-gates are selected based on one or more optical properties of said object.

6. The method as claimed in any one of claims 3 or 4 wherein said initial time and said final time of said selected time-gates are estimated based on one or more optical properties of said object that influence propagation of said light within said object.

7. The method according to claim 6 wherein said one or more properties comprise a thickness of said object.

8. The method as claimed in any one of claims 1 - 7 wherein said step of detecting is performed using a time-gated detector.

9. The method as claimed in any one of claims 1 - 7 wherein two or more of said first time-gates are simultaneously detected at two or more time-gated detectors having a synchronized acquisition time gate.

10. The method as claimed in any one of claims 1 - 8 wherein the step of simultaneously detecting comprises detecting said selected time-gates using a time-gated detector comprising a two-dimensional array of pixels.

11. The method as claimed in any one of claims 8 or 9 wherein the time-gated detector is an intensified charge coupled device camera.

12. The method as claimed in any one of claims 1 - 11 wherein the collecting of the light is achieved by providing one or more optical fibers.

13. The method as claimed in claim 12 further comprising adjusting fiber length to introduce the desired delays.

14. The method as claimed in claim 13 wherein the fibers are grouped together into one or more bundles.

15. The method according to claim 14 wherein each fiber in the one or more bundles is directed to a distinct detection position of the time-gated detector or to a distinct time-gated detector.

16. The method as claimed in claim 15 wherein the one or more bundles are spatially localized such as to collect light from one or more desired areas of said object.

17. The method as claimed in claim 16 wherein the one or more bundles are coupled to one or more time-gated detectors.

18. A system for optical imaging of a light scattering object, the system comprising:
i) at least one light injection port (14);
ii) light collecting means (18) to collect light from said object at at least two collection ports to provide at least two optical signal based temporal point spread functions; and
iii) means (30) for introducing temporal delays in propagation of said optical signals;
iv) at least two time-gated detectors (22);
**characterized in that** said at least two time-gated detectors are constructed to detect at least a first time gate of each temporal point spread function wherein each of said first time gates is obtained from a different time delayed temporal point spread function, and wherein all of said first time gates are simultaneously detected.

19. The system as claimed in claim 18 wherein said two or more collection ports are proximal and said temporal point spread functions are substantially identical.

20. The system as claimed in claim 18 or 19 wherein the light collecting means are optical fibers.

21. The system as claimed in claim 20 wherein the optical fibers are also the means to delay the propagation of the optical signals, whereby the delay is provided by having optical fibers of different lengths.

## Patentansprüche

1. Verfahren zum Reduzieren der Bildgewinnungszeit beim optischen Abbilden eines lichtstreuenden Objektes, wobei das Verfahren die folgenden Schritte umfaßt:
i) Einleiten eines Lichtpulses an einem Einleitungsanschluß in das Objekt zu einer Zeit t₀,
ii) Auffangen von Licht von dem Objekt an wenigstens zwei Auffanganschlüssen um eine Vielzahl von auf einem optischen Signal basierenden zeitlichen Punktverteilungsfunktionen zur Verfügung zu stellen, und
iii) Erfassen wenigstens eines ersten Zeitfensters jeder zeitlichen Punktverteilungsfunktion um beim Erstellen eines optischen Bildes des lichtstreuenden Objektes zu verwendende Informationen zur Verfügung zu stellen,
**dadurch gekennzeichnet,**
**daß** gewünschte zeitliche Verzögerungen in den Ausbreitungsweg der optischen Signale eingeführt werden, um zeitverzögerte zeitliche Punktverteilungsfunktionen zu erzeugen, wobei jedes der ersten Zeitfenster von einer anderen zeitverzögerten zeitlichen Punktverteilungsfunktion erhalten wird und wobei alle ersten Zeitfenster simultan erfaßt werden.

2. Verfahren nach Anspruch 1, wobei die zwei oder mehr Auffanganschlüsse proximal sind und wobei die zeitlichen Punktverteilungsfunktionen im wesentlichen identisch sind.

3. Verfahren nach Anspruch 1 oder 2, wobei die Zeitfenster ein Zeitinterwal umfassen, das durch eine Anfangszeit und eine Endzeit definiert ist, die relativ zu t₀ gewählt werden.

4. Verfahren nach Anspruch 3, wobei die ausgewählten Zeitfenster für eine Vielzahl von Einleitungsanschluß-Objekt-Auffanganschluß-Geometrien verwendet werden.

5. Verfahren nach einem der Ansprüche 1-4, wobei die Zeitfenster basierend auf einer oder mehreren optischen Eigenschaft(en) des Objektes ausgewählt werden.

6. Verfahren nach einem der Ansprüche 3 oder 4, wobei die Anfangszeit und die Endzeit der ausgewählten Zeitfenster basierend auf einer oder mehreren optischen Eigenschaft(en) des Objektes, die die Ausbreitung des Lichtes innerhalb des Objektes beeinflußt/beeinflussen, abgeschätzt werden.

7. Verfahren nach Anspruch 6, wobei die eine oder die mehreren Eigenschaft(en) eine Dicke des Objektes umfassen.

8. Verfahren nach einem der Anspruch 1-7, wobei der Schritt des Erfassens unter Verwendung eines Detektors mit Zeitfenster ausgeführt wird.

9. Verfahren nach einem der Anspruch 1-7, wobei zwei oder mehrere der ersten Zeitfenster simultan mit zwei oder mehr Detektoren mit Zeitfenstern, die ein synchronisiertes Gewinnungszeitfenster besitzen, erfaßt werden.

10. Verfahren nach einem der Anspruch 1-8, wobei der Schritt des simultanen Erfassens das Erfassen der ausgewählten Zeitfenster unter Verwendung eines Detektors mit Zeitfenster umfaßt, der ein zweidimensionales Pixelarray umfaßt.

11. Verfahren nach einem der Ansprüche 8 oder 9, wobei der Detektor mit Zeitfenster eine verstärkte CCD-Kamera ist.

12. Verfahren nach einem der Ansprüche 1-11, wobei das Auffangen des Lichtes durch Vorsehen von einer oder mehreren optischen Faser(n) erreicht wird.

13. Verfahren nach Anspruch 12, ferner umfassend das Einstellen der Faserlänge um die gewünschten Verzögerungen einzuführen.

14. Verfahren nach Anspruch 13, wobei die Fasern in einem oder mehreren Bündel(n) gruppiert sind.

15. Verfahren nach Anspruch 14, wobei jede Faser in dem einen oder den mehreren Bündel(n) auf eine bestimmte Erfassungsposition des Detektors mit Zeitfenster oder auf einen bestimmten Detektor mit Zeitfenster gerichtet ist.

16. Verfahren nach Anspruch 15, wobei das eine oder die mehreren Bündel räumlich lokalisiert sind, um Licht aus einem oder mehreren gewünschten Bereich(en) des Objektes aufzufangen.

17. Verfahren nach Anspruch 16, wobei das eine oder die mehreren Bündel mit einem oder mehreren Detektor(en) mit Zeitfenster gekoppelt sind.

18. System zum optischen Abbilden eines Lichtstreuenden Objektes, wobei das System umfaßt:
i) wenigstens einen Lichteinleitungsanschluß (14),
ii) Lichtauffangmittel (18) zum Auffangen von Licht von dem Objekt an wenigstens zwei Auffanganschlüssen, um wenigstens zwei auf optischen Signalen basierende zeitliche Punktverteilungsfunktionen zur Verfügung zu stellen,
iii) Mittel (30) zum Einführen eines Zeitverzuges in die Ausbreitung der optischen Signale,
iv) wenigstens zwei Detektoren (22) mit Zeitfenster,
**dadurch gekennzeichnet**,
das die wenigstens zwei Detektoren mit Zeitfenster dazu konstruiert sind, wenigstens ein erstes Zeitfenster jeder zeitlichen Punktverteilungsfunktion zu erfassen, wobei jedes der ersten Zeitfenster von einer um unterschiedliche Zeiten verzögerten zeitlichen Punktverteilungsfunktion erhalten wird und wobei alle ersten Zeitfenster simultan erfaßt werden.

19. System nach Anspruch 18, wobei die beiden oder mehr Auffanganschlüsse proximal sind und wobei die zeitlichen Punktverteilungsfunktionen im wesentlichen identisch sind.

20. System nach Anspruch 18 oder 19, wobei die Lichtauffangmittel optische Fasern sind.

21. System nach Anspruch 20, wobei die optischen Fasern auch die Mittel zum Verzögern der Ausbreitung der optischen Signale darstellen, wobei die Verzögerung **dadurch** erzeugt wird, daß die optischen Fasern verschiedene Längen besitzen.

## Revendications

1. Procédé de réduction du temps d'acquisition d'une image lors de la mise en image optique d'un objet diffusant de la lumière, le procédé comprenant les étapes consistant à :
i) injecter une impulsion de lumière au niveau d'un port d'injection dans ledit objet à un temps t₀ ;
ii) collecter la lumière provenant dudit objet au niveau d'au moins deux ports de collecte pour fournir une pluralité de fonctions d'étalement de point temporel basées sur un signal optique ; et
iii) détecter au moins une première passerelle temporelle de chaque dite fonction d'étalement de point temporel pour fournir des informations à utiliser dans la production d'une image optique dudit objet diffusant de la lumière ;
**caractérisé en ce que** les retards temporels souhaités sont introduits dans le chemin de propagation des signaux optiques pour produire des fonctions d'étalement de point temporel temporisées, dans lesquelles chacune desdites premières passerelles temporelles est obtenue à partir d'une fonction d'étalement de point temporel temporisée différente, et dans lesquelles toutes lesdites premières passerelles temporelles sont détectées simultanément.

2. Procédé selon la revendication 1, dans lequel lesdits au moins deux ports de collecte sont proximaux et lesdites fonctions d'étalement de point temporel sont sensiblement identiques.

3. Procédé selon l'une quelconque des revendications 1 à 2, dans lequel lesdites passerelles temporelles couvrent un intervalle de temps défini par un temps initial et un temps final qui sont définis par rapport à t₀.

4. Procédé selon la revendication 3, dans lequel lesdites passerelles temporelles sélectionnées sont utilisées pour une pluralité de géométries de port d'injection/objet/port de détecteur.

5. Procédé selon l'une quelconque des revendications 1 à 4, dans lequel lesdites passerelles temporelles sont sélectionnées en fonction d'une ou plusieurs propriétés optiques dudit objet.

6. Procédé selon l'une quelconque des revendications 3 ou 4 dans lequel ledit temps initial et ledit temps final desdites passerelles temporelles sélectionnées sont estimées en fonction d'une ou plusieurs propriétés optiques dudit objet qui influencent la propagation de ladite lumière dans ledit objet.

7. Procédé selon la revendication 6, dans lequel lesdites une ou plusieurs propriétés comprennent une épaisseur dudit objet.

8. Procédé selon l'une quelconque des revendications 1 à 7, dans lequel ladite étape de détection est réalisée à l'aide d'un détecteur à passerelle temporelle.

9. Procédé selon l'une quelconque des revendications 1 à 7, dans lequel deux ou plus desdites premières passerelles temporelles sont détectées simultanément au niveau de deux détecteurs à passerelle temporelles ou plus ayant une passerelle temporelle d'acquisition synchronisée.

10. Procédé selon l'une quelconque des revendications 1 à 8, dans lequel l'étape de détection simultanée comprend la détection desdites passerelles temporelles sélectionnées à l'aide d'un détecteur à passerelle temporelle comprenant un réseau bidimensionnel de pixels.

11. Procédé selon l'une quelconque des revendications 8 ou 9, dans lequel le détecteur à passerelle temporelle est une caméra de dispositif à couplage de charge intensifié.

12. Procédé selon l'une quelconque des revendications 1 à 11, dans lequel la collecte de la lumière est obtenue en fournissant une ou plusieurs fibres optiques.

13. Procédé selon la revendication 12, comprenant en outre l'étape consistant à ajuster la longueur de fibre pour introduire les retards souhaités.

14. Procédé selon la revendication 13, dans lequel les fibres sont groupées ensemble dans un ou plusieurs faisceaux.

15. Procédé selon la revendication 14, dans lequel chaque fibre dans ledit un ou plusieurs faisceaux est dirigée vers une position de détection distincte du détecteur à passerelle temporelle ou vers un détecteur à passerelle temporelle distinct.

16. Procédé selon la revendication 15, dans lequel lesdits un ou plusieurs faisceaux sont localisés dans l'espace de manière à collecter la lumière en provenance d'une ou plusieurs zones souhaitées dudit objet.

17. Procédé selon la revendication 16, dans lequel lesdits un ou plusieurs faisceaux sont couplés à un ou plusieurs détecteurs à passerelle temporelle.

18. Système de mise en image optique d'un objet diffusant de la lumière, le système comprenant :
i) au moins un port d'injection de lumière (14) ;
ii) des moyens de collecte de lumière (18) pour collecter la lumière provenant dudit objet au niveau d'au moins deux ports de collecte pour fournir au moins deux fonctions d'étalement de point temporel basées sur un signal optique ; et
iii) des moyens (30) destinés à introduire des retards temporels dans la propagation desdits signaux optiques ;
iv) au moins deux détecteurs à passerelle temporelle (22) ;
**caractérisé en ce que** lesdits au moins deux détecteurs à passerelle temporelle sont construits pour détecter au moins une première passerelle temporelle de chaque fonction d'étalement de point temporel dans lequel chacune desdites premières passerelles temporelles est obtenue à partir d'une fonction d'étalement de point temporel temporisée différente, et dans lequel toutes lesdites passerelles temporelles sont détectées simultanément.

19. Système selon la revendication 18, dans lequel lesdits deux ports de collecte ou plus sont proximaux et lesdites fonctions d'étalement de point temporel sont sensiblement identiques.

20. Système selon la revendication 18 ou 19 dans lequel les moyens de collecte de lumière sont des fibres optiques.

21. Système selon la revendication 20 dans lequel les fibres optiques sont également les moyens pour retarder la propagation des signaux optiques, moyennant quoi le retard est fourni en ayant des fibres optiques de différentes longueurs.
